# EUROPEAN PATENT APPLICATION

(11) **EP 4 527 336 A1**
(43) Date of publication of application: **26.03.2025**
(21) Application number: 22943587.0
(22) Date of filing: 21.12.2022
(51) Int. Cl.: A61B 34/30, A61B 17/00

(54) **OPERATING SYSTEM AND METHOD FOR ERCP SURGICAL ROBOT**

(30) Priority: 23.05.2022 CN 202210562085
(71) Applicant: Shanghai Operation Robot Co., Ltd., Shanghai 201318 (CN)
(72) Inventor: YU, Zhongwei, Shanghai 201318 (CN); LIU, Wenming, Shanghai 201318 (CN); SUN, Yucheng, Shanghai 201318 (CN); XU, Changkai, Shanghai 201318 (CN); YANG, Xiaoyi, Shanghai 201318 (CN); DI, Xiaotao, Shanghai 201318 (CN); ZHANG, Yadong, Shanghai 201318 (CN); LIU, Daozhi, Shanghai 201318 (CN); LIU, Yikun, Shanghai 201318 (CN)
(74) Representative: Bayramoglu et al.
(86) International application number: PCT/CN2022/140593
(87) International publication number: WO 2023/226400

(57) **Abstract**

An operating system and method for an endoscopic retrograde cholangiopancreatography (ERCP) surgical robot, including: an operating table (1), where a patient lies on the operating table (1); an endoscopic actuation end device (2); an endoscopic accessory operation end device (3); a console (4), including an operating handle (41), a foot switch (42), and a three-dimensional (3D) handle (43); and a display screen (5) for human-computer interaction. A forceps lifter control module (21), a knob control module (22), a catheter drive module (23) and a water vapor suction control module (24) on the endoscopic actuation end device (2) are controlled through the operating handle (41), the foot switch (42), the 3D handle (43) and the touch screen (44) on the console (4). A guide wire drive module (31) on the endoscopic accessory operation end device (3) remotely controls the insertion and feeding of a guide wire or catheter to complete the surgery. The present disclosure reduces the harm caused by long-term surgical radiation to operators and avoids hand shaking of doctors during operation. The operating handle (41) is configured as an endoscope operating part structure, which facilitates doctors to adapt to the handle control method with minimal learning and improves the convenience of use.

## Description

### TECHNICAL FIELD

The present disclosure relates to the technical field of medical appliances, and specifically to an operating system and method for an endoscopic retrograde cholangiopancreatography (ERCP) surgical robot.

### BACKGROUND TECHNOLOGY

Endoscopic retrograde cholangiopancreatography (ERCP) is a very mature minimally invasive endoscopic surgery for the treatment of biliary-pancreatic system diseases. ERCP can be used for the diagnosis and treatment of diseases such as gallstones, biliary obstruction, cholangitis, biliary tumors, and pancreatic tumors. During the surgery, a duodenoscope is inserted into the descending part of the patient's duodenum, and a contrast catheter is inserted into the opening of the duodenal papilla through a biopsy channel to inject the contrast agent. The specific condition of the pancreatic and biliary ducts is observed under X-rays to determine whether there is a lesion before corresponding surgery is performed. ERCP has the advantages of minimal trauma, short operation time, few complications, and high safety, etc. This type of surgery involves minimal surgical trauma without causing too much pain to patients, and features fast postoperative recovery.

At present, ERCP in China is manually performed by doctors and their teams. Due to the fact that ERCP is performed with the assistance of X-rays, the surgeon is exposed to X-rays for a long time. During surgery, although operators need to wear thick radiation protection clothing, their arms are exposed to operate and thus unprotected. Years of surgical radiation will cause serious radiation injuries to operators.

Currently, ERCP requires a lot of operators and collaborators, making the operating room with limited space appear congested. Doctors and operators need to stand all day, with high work intensity and easy fatigue, which may affect the accuracy of surgery and even lead to errors. During the surgery, after the duodenoscope is inserted into the body, the angle of the guide wire catheter inside the body needs to be adjusted using a forceps lifter. However, it is hard for doctors and operators to prevent their hands from shaking, so the instrument inserted into the body often shifts after being positioned, making it impossible to accurately adjust the forceps lifter. Meanwhile, if the forceps lifter is directly adjusted, the operator will be exposed to X-rays, which will cause harm to the operator.

Chinese patent with publication number of CN213075919U discloses a novel ERCP surgical instrument table, which belongs to the field of medical technology. The novel ERCP surgical instrument table includes a support base. A plurality of connecting rods are vertically and fixedly provided at four corners at the top of the support base. A box is supported and placed between the plurality of connecting rods. A front surface of the box is provided with a plurality of slots. Inner wall surfaces of the plurality of slots are fixedly provided with drawer grooves. A plurality of drawers are slidably provided on the surfaces of the drawer grooves. Universal wheels are fixedly provided at four corners at the bottom of the support base.

The instrument table in the prior art is placed in the operating room for doctors to access instruments, making it hard to reduce the harm caused by long-term surgical radiation to operators and avoid the hand shaking of doctors during operation. Therefore, there are areas for improvement in the prior art.

### CONTENT OF THE INVENTION

In view of the defects in the prior art, an objective of the present disclosure is to provide an operating system and method for an endoscopic retrograde cholangiopancreatography (ERCP) surgical robot.

According to one or more embodiments of the present disclosure, an operating system for an ERCP surgical robot includes: an operating table, where a patient lies on the operating table; an endoscopic actuation end device, including an endoscopic actuation end operating platform with a forceps lifter control module, a knob control module, a catheter drive module and a water vapor suction control module, and an auxiliary structure for adjusting a position and a posture of an endoscope; an endoscopic accessory operation end device, including an accessory operating platform provided with a guide wire drive module and configured to drive a guide wire for an advancing, retracting or locking operation; a console, including an operating handle, where the operating handle is configured to control the forceps lifter control module, the knob control module and the water vapor suction control module; a foot switch and a three-dimensional (3D) handle, where the foot switch is configured to switch between driving functions of the catheter drive module and the guide wire drive module, and the 3D handle is configured to provide push/pull force feedback of the catheter drive module or the guide wire drive module; and a display screen for human-computer interaction.

Further, the auxiliary structure includes a drag arm and a U-shaped arm, where the drag arm is a multi-degree-of-freedom (MDOF) mechanical arm, and the U-shaped arm is suspended at an actuation end of the drag arm.

Further, the endoscopic accessory operation end device further includes a collaborative arm, and the accessory operating platform is provided at an actuation end of the collaborative arm.

Further, each of the endoscopic actuation end device and the endoscopic accessory operation end device includes a trolley.

Further, the structure of the operating handle is a structure for simulating an endoscope operating part.

Further, a side of the operating table is provided with an X-ray machine; the X-ray machine is configured to scan the patient on the operating table; and a scanning result of the X-ray machine is synchronized to the display screen.

Further, the console is further provided with a touch screen, and the touch screen is configured to control the endoscopic actuation end device and the endoscopic accessory operation end device.

Further, the operating table, the endoscopic actuation end device and the endoscopic accessory operation end device are all located in an operating room, and the console is located outside the operating room.

Further, the console is configured to control the endoscopic actuation end device and the endoscopic accessory operation end device through an Ethernet for control automation technology (EtherCT) bus.

According to the present disclosure, an operating method for an ERCP surgical robot includes the following steps: S1: preforming machine resetting, and asking the patient to lie on the operating table; S2: inserting an insertion part of a duodenoscope into a duodenum of the patient through the patient's mouth, esophagus and stomach; dragging the U-shaped arm to the patient's head; mounting a handle of the endoscope operating part into an endoscope base; and fixing a position of the U-shaped arm; S3: inserting a guide wire into a catheter; inserting the catheter into the duodenum; and mounting a catheter body into the catheter drive module; S4: adjusting, by the collaborative arm, the accessory operating platform to an appropriate position, and fixing the accessory operating platform; S5: placing the operating handle of the catheter on the accessory console, inserting the guide wire into a hose on the console, and connecting the hose to a guide wire outlet of the catheter, thereby fixing the catheter; S6: mounting the guide wire into the guide wire drive module, thereby completing surgical preparation work; and S7: remotely controlling, by a doctor sitting at the console, the insertion and feeding of the guide wire or the catheter through the operating handle, the foot switch, the 3D handle and the touch screen.

### DESCRIPTION OF THE DRAWINGS

Other features, objectives, and advantages of the present disclosure will become more apparent by reading the detailed description of non-limiting embodiments with reference to the following drawings.
FIG. 1 is an overall structural diagram of a surgical robot operating device according to an embodiment of the present disclosure; and
FIG. 2 is a logic block diagram of a console control system according to an embodiment of the present disclosure.

### Reference Signs:

| | | | |
|---|---|---|---|
| 1. | operating table | 32. | collaborative arm |
| 2. | endoscopic actuation end device | 4. | console |
| 21. | forceps lifter control module | 41. | operating handle |
| 22. | knob control module | 42. | foot switch |
| 23. | catheter drive module | 43. | 3D handle |
| 24. | water vapor suction control module | 44. | touch screen |
| 25. | drag arm | 5. | display screen |
| 26. | U-shaped arm | 6. | trolley |
| 3. | endoscopic accessory operation end device | 7. | X-ray machine |
| 31. | guide wire drive module | | |

### SPECIFIC IMPLEMENTATIONS

The present disclosure is described in detail below with reference to specific embodiments. The following embodiments will help those skilled in the art to further understand the present disclosure, but do not limit the present disclosure in any way. It should be noted that several variations and improvements can also be made by a person of ordinary skill in the art without departing from the conception of the present disclosure. These all fall within the protection scope of the present disclosure.

### Embodiment 1

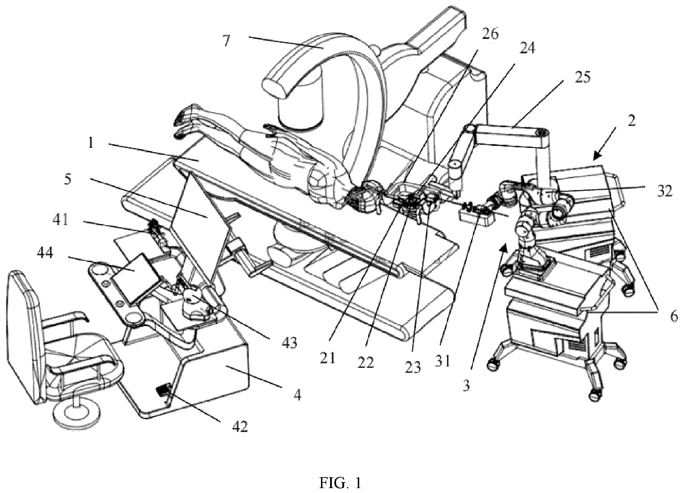

As shown in FIG. 1, an embodiment of the present disclosure provides an operating system for an ERCP surgical robot, including operating table 1, endoscopic actuation end device 2, endoscopic accessory operation end device 3, console 4, and display screen 5 for human-computer interaction. The endoscopic actuation end device 2 includes an endoscopic actuation end operating platform with forceps lifter control module 21, knob control module 22, catheter drive module 23 and water vapor suction control module 24, and includes an auxiliary structure for adjusting a position and a posture of an endoscope. The endoscopic accessory operation end device 3 includes an accessory operating platform provided with guide wire drive module 31 and configured to drive a guide wire for an advancing, retracting or locking operation.

The console 4 includes operating handle 41, foot switch 42, and three-dimensional (3D) handle 43. The operating handle 41 is configured to control the forceps lifter control module 21, the knob control module 22 and the water vapor suction control module 24. The structure of the operating handle 41 is a structure for simulating an endoscope operating part, which is convenient for doctors to operate. The foot switch 42 is configured to switch between driving functions of the catheter drive module 23 and the guide wire drive module 31. The 3D handle 43 is configured to provide push/pull force feedback of the catheter drive module 23 or the guide wire drive module 31. The catheter drive module 23 and the guide wire drive module 31 both provide push/pull force feedback to the 3D handle 43, and doctors can make judgments and reactions based on the magnitude of the force.

The auxiliary structure includes drag arm 25 and U-shaped arm 26. The drag arm 25 is a multi-degree-of-freedom (MDOF) mechanical arm. The U-shaped arm 26 is suspended at an actuation end of the drag arm 25. The U-shaped arm 26 is connected to a cantilever at the actuation end of the drag arm 25 through a connecting rod, and the cantilever at the actuation end of the drag arm 25 is connected to a fastener. The U-shaped arm 26 is provided with a control handle. The U-shaped arm 26 is fixedly provided with a duodenoscope. Under normal conditions, the U-shaped arm 26 and the actuation end of the drag arm 25 are relatively fixed. When it is necessary to adjust the position and posture of the U-shaped arm 26, the control handle is pressed and held. A photoelectric sensor of the control handle sends an unlock signal, and the fastener on the cantilever is released. In this way, the user can move the U-shaped arm 26 and an endoscope base within a certain range. The endoscopic actuation end device 2 includes trolley 6. The drag arm 25 is provided on the trolley 6 to move the endoscopic actuation end device 2.

As shown in FIG. 1, the endoscopic accessory operation end device 3 further includes collaborative arm 32. The accessory operating platform is provided at an actuation end of the collaborative arm 32. The guide wire drive module 31 is provided on the accessory operating platform. The endoscopic accessory operation end device 3 further includes trolley 6. The collaborative arm 32 is provided on the trolley 6 to move the endoscopic accessory operation end device 3.

A side of the operating table 1 is provided with X-ray machine 7. The X-ray machine 7 is configured to scan a patient on the operating table 1. A scanning result of the X-ray machine 7 is synchronized to the display screen 5. In the present disclosure, the X-ray machine 7 is preferably a C-arm X-ray machine.

The console 4 controls the endoscopic actuation end device 2 and the endoscopic accessory operation end device 3 through an EtherCT bus. The console 4 is further provided with touch screen 44, and the touch screen 44 is configured to control the endoscopic actuation end device 2 and the endoscopic accessory operation end device 3. The doctor can control the endoscopic actuation end device 2 and the endoscopic accessory operation end device 3 through the operating handle 41, the foot switch 42, and the 3D handle 43 during operation. The doctor can also control the endoscopic actuation end device 2 and the endoscopic accessory operation end device 3 through the touch screen 44.

In the present disclosure, the operating table 1, the endoscopic actuation end device 2 and the endoscopic accessory operation end device 3 are all located in the operating room, and the console 4 is located outside the operating room. Doctors can operate remotely outside the operating room to reduce the harm caused by long-term surgical radiation to operators and avoid hand shaking during operation.

### Embodiment 2

Based on Embodiment 1, an embodiment of the present disclosure provides an operating method for an ERCP surgical robot. The operating method adopts the above operating system for an ERCP surgical robot, and includes the following steps.

S1. Machine resetting is performed, and a patient lies on the operating table 1.

S2. An insertion part of the duodenoscope is inserted into a duodenum of the patient through the patient's mouth, esophagus and stomach. The U-shaped arm 26 is dragged to the patient's head. A handle of the endoscope operating part is mounted into an endoscope base. A position of the U-shaped arm 26 is fixed. The insertion part of the duodenoscope is a flexible tube part. The drag arm 25 is unlocked by pressing the control handle, and the U-shaped arm 26 is dragged to the patient's head. A position is adjusted, and a control handle switch is released to fix the U-shaped arm 26.

S3. A guide wire is inserted into a catheter, and the catheter is inserted into the duodenum via a through-the-scope (TTS) opening. After insertion in place, the catheter is put into a guide slot of the U-shaped arm 26 and covered by a guide slot cover. A catheter body is placed into the catheter drive module 23.

S4. The collaborative arm 32 adjusts the accessory operating platform to an appropriate position, such that the accessory operating platform is fixed. An adjustment button on the collaborative arm 32 is pressed and held to adjust the accessory operating platform to an appropriate position. Then the button is released, and the accessory operating platform is fixed.

S5. The operating handle 41 of the catheter is placed on the accessory console 4. The guide wire is inserted into a hose on the console 4, and the hose is connected to a guide wire outlet of the catheter, thereby fixing the catheter.

S6. The guide wire is mounted into the guide wire drive module 31 to achieve the fixation or delivery of the guide wire, thereby completing surgical preparation work.

S7. The doctor sits at the console 4 and remotely controls the insertion and feeding of the guide wire or the catheter through the operating handle 41, the foot switch 42, the 3D handle 43 and the touch screen 44. In this way, a series of surgeries such as balloon dilation, stone removal, stent placement, and drainage are completed.

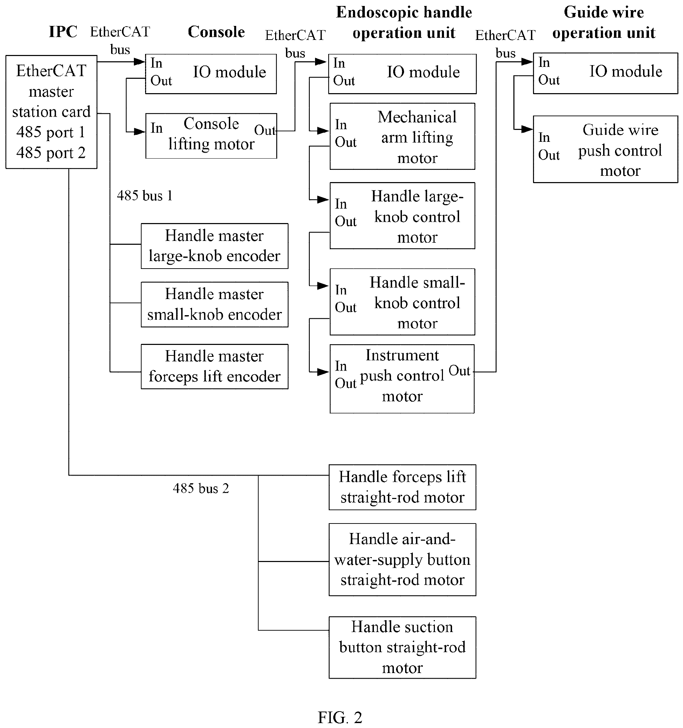

As shown in FIG. 2, a control system of the console 4 is a Linux system, which specifically includes an inter-process communication (IPC) module, a console 4 control module, an endoscopic handle operation unit, and a guide wire operation unit.

The IPC module includes an EtherCAT master station card, 485 port 1, and 485 port 2. The IPC module is connected to a handle forceps lifter straight-rod motor, a handle air-and-water-supply button straight-rod motor, and a handle suction button straight-rod motor through 485 bus 2. The IPC module is further connected to a handle master large-knob encoder, a handle master small-knob encoder, and a handle master forceps lifter encoder through 485 bus 2.

The console 4 includes an input/output (IO) module and a console 4 lifting motor. The IPC module is connected to an IN terminal of the IO module of the console 4 through an EtherCAT bus. An OUT terminal of the IO module of the console 4 is connected to an IN terminal of the console 4lifting motor.

The endoscopic handle operation unit includes an IO module, a mechanical arm lifting motor, a handle large-knob control motor, a handle small-knob control motor, and an instrument push control motor. The IO module of the endoscopic handle operation unit is connected to an OUT terminal of the console 4 lifting motor. An OUT terminal of the IO module of the endoscopic handle operation unit is connected to an IN terminal of a mechanical arm lifting motor. An OUT terminal of the mechanical arm lifting motor is connected to an IN terminal of the handle large-knob control motor. An OUT terminal of the handle large-knob control motor is connected to an IN terminal of the handle small-knob control motor. An OUT terminal of the handle small-knob control motor is connected to an IN terminal of the instrument push control motor.

The guide wire operation unit includes an IO module and a guide wire push control motor. An IN terminal of the IO module of the guide wire operation unit is connected to an OUT terminal of the instrument push control motor. An OUT terminal of the IO module of the guide wire operation unit is connected to an IN terminal of the guide wire push control motor.

### Working Principle

During surgery, first, machine resetting is performed, and the patient is asked to lie on the operating table 1. Then, the insertion part of the duodenoscope is inserted into a duodenum of the patient through the patient's mouth, esophagus and stomach. The U-shaped arm 26 is dragged to the patient's head. The handle of the endoscope operating part is mounted into the endoscope base, and the position of the U-shaped arm 26 is fixed. Then, the guide wire is inserted into the catheter. The catheter is inserted into the duodenum, and the catheter body is mounted into the catheter drive module 23. The accessory operating platform is adjusted to an appropriate position through the collaborative arm 32, and is then fixed. The operating handle 41 of the catheter is placed on the accessory console 4. The guide wire is inserted into the hose on the console 4, and the hose is connected to the guide wire outlet of the catheter, thereby fixing the catheter. The guide wire is mounted into the guide wire drive module 31, and the surgical preparation work is completed. Finally, the doctor sits at the console 4 and remotely controls the insertion and feeding of the guide wire or the catheter through the operating handle 41, the foot switch 42, the 3D handle 43 and the touch screen 44.

Those skilled in the art are aware that in addition to being realized by using pure computer-readable program code, the system and each apparatus, module, and unit thereof provided in the present disclosure can realize a same program in a form of a logic gate, a switch, an application-specific integrated circuit, a programmable logic controller, or an embedded microcontroller by performing logic programming on the method steps. Therefore, the system and each apparatus, module, and unit thereof provided in the present disclosure can be regarded as a kind of hardware component. The apparatus, module, and unit included therein for realizing each function can also be regarded as a structure in the hardware component; and the apparatus, module, and unit for realizing each function can also be regarded as a software module for implementing the method or a structure in the hardware component.

In the description of the present disclosure, it needs to be understood the orientation or positional relationships indicated by terms, such as "up", "down", "front", "rear", "left", "right", "vertical", "horizontal", "top", "bottom", "inside", and "outside", are based on the orientation or positional relationship shown in the drawings, are merely for facilitating the description of the present disclosure and simplifying the description, rather than indicating or implying that an apparatus or element referred to must have a particular orientation or be constructed and operated in a particular orientation, and therefore shall not be interpreted as limiting the present disclosure.

Compared with the prior art, the present disclosure has the following beneficial effects:
1. In the present disclosure, the forceps lifter control module, the knob control module, the catheter drive module and the water vapor suction control module on the endoscopic actuation end device are controlled through the operating handle, the foot switch, the 3D handle and the touch screen on the console. The guide wire drive module on the endoscopic accessory operation end device remotely controls the insertion and feeding of the guide wire or the catheter to complete a series of surgeries such as balloon dilation, stone removal, stent placement, and drainage. The present disclosure reduces the harm caused by long-term surgical radiation to operators and avoids hand shaking of doctors during operation.
2. In the present disclosure, the shape of the operating handle is configured as an endoscope operating part structure, which facilitates doctors to adapt to the handle control method with minimal learning process and improves the convenience of using the operating system of the surgical robot.
3. In the present disclosure, the scanning results of the X-ray machine are synchronized to the display screen, which improves the accuracy of the doctor's operation and thus increases the success rate of the surgery.

The specific embodiments of the present disclosure are described above. It should be understood that the present disclosure is not limited to the above specific implementations, and a person skilled in the art can make various variations or modifications within the scope of the claims without affecting the essence of the present disclosure. The embodiments of the present disclosure and features in the embodiments may be arbitrarily combined with each other in a non-conflicting situation.

## Claims

1. An operating system for an endoscopic retrograde cholangiopancreatography (ERCP) surgical robot, **characterized by** comprising:
an operating table (1), wherein a patient lies on the operating table (1);
an endoscopic actuation end device (2), comprising an endoscopic actuation end operating platform with a forceps lifter control module (21), a knob control module (22), a catheter drive module (23) and a water vapor suction control module (24), and an auxiliary structure for adjusting a position and a posture of an endoscope;
an endoscopic accessory operation end device (3), comprising an accessory operating platform provided with a guide wire drive module (31) and configured to drive a guide wire for an advancing, retracting or locking operation;
a console (4), comprising an operating handle (41), wherein the operating handle (41) is configured to control the forceps lifter control module (21), the knob control module (22) and the water vapor suction control module (24);
a foot switch (42) and a three-dimensional (3D) handle (43), wherein the foot switch (42) is configured to switch between driving functions of the catheter drive module (23) and the guide wire drive module (31), and the 3D handle (43) is configured to provide push/pull force feedback of the catheter drive module (23) or the guide wire drive module (31); and
a display screen (5) for human-computer interaction.

2. The operating system for the ERCP surgical robot according to claim 1, **characterized in that** the auxiliary structure comprises a drag arm (25) and a U-shaped arm (26), wherein the drag arm (25) is a multi-degree-of-freedom (MDOF) mechanical arm, and the U-shaped arm (26) is suspended at an actuation end of the drag arm (25).

3. The operating system for the ERCP surgical robot according to claim 1, **characterized in that** the endoscopic accessory operation end device (3) further comprises a collaborative arm (32), and the accessory operating platform is provided at an actuation end of the collaborative arm (32).

4. The operating system for the ERCP surgical robot according to claim 1, **characterized in that** each of the endoscopic actuation end device (2) and the endoscopic accessory operation end device (3) comprises a trolley (6).

5. The operating system for the ERCP surgical robot according to claim 1, **characterized in that** a structure of the operating handle (41) is a structure for simulating an endoscope operating part.

6. The operating system for the ERCP surgical robot according to claim 1, **characterized in that** a side of the operating table (1) is provided with an X-ray machine (7); the X-ray machine (7) is configured to scan the patient on the operating table (1); and a scanning result of the X-ray machine (7) is synchronized to the display screen (5).

7. The operating system for the ERCP surgical robot according to claim 1, **characterized in that** the console (4) is further provided with a touch screen (44), and the touch screen (44) is configured to control the endoscopic actuation end device (2) and the endoscopic accessory operation end device (3).

8. The operating system for the ERCP surgical robot according to claim 1, **characterized in that** the operating table (1), the endoscopic actuation end device (2) and the endoscopic accessory operation end device (3) are all located in an operating room, and the console (4) is located outside the operating room.

9. The operating system for the ERCP surgical robot according to claim 1, **characterized in that** the console (4) is configured to control the endoscopic actuation end device (2) and the endoscopic accessory operation end device (3) through an Ethernet for control automation technology (EtherCT) bus.

10. An operating method for an ERCP surgical robot, **characterized by** being implemented through the operating system for the ERCP surgical robot according to any one of claims 1 to 6, and comprising the following steps:
S1: preforming machine resetting, and asking the patient to lie on the operating table (1);
S2: inserting an insertion part of a duodenoscope into a duodenum of the patient through the patient's mouth, esophagus and stomach; dragging a U-shaped arm (26) to the patient's head; mounting a handle of an endoscope operating part into an endoscope base; and fixing a position of the U-shaped arm (26);
S3: inserting the guide wire into a catheter; inserting the catheter into the duodenum; and mounting a catheter body into the catheter drive module (23);
S4: adjusting, by a collaborative arm (32), the accessory operating platform to an appropriate position, and fixing the accessory operating platform;
S5: placing the operating handle (41) of the catheter on the accessory console (4), inserting the guide wire into a hose on the console (4), and connecting the hose to a guide wire outlet of the catheter, thereby fixing the catheter;
S6: mounting the guide wire into the guide wire drive module (31), thereby completing surgical preparation work; and
S7: remotely controlling, by a doctor sitting at the console (4), an insertion and a feeding of the guide wire or the catheter through the operating handle (41), the foot switch (42), the 3D handle (43) and the touch screen (44).
